(19) Europäisches Patentamt
European Patent Office
Office européen des brevets

(11) Publication number: **0 319 614 A1**

# (12) EUROPEAN PATENT APPLICATION

(21) Application number: **87202434.4**

(22) Date of filing: **07.12.87**

(51) Int. Cl.4: **C08F 8/28 , C08F 8/12 , C07C 47/04**

(43) Date of publication of application: **14.06.89 Bulletin 89/24**

(84) Designated Contracting States: **AT BE CH DE ES FR GB GR IT LI LU NL SE**

(71) Applicant: **METHANOL CHEMIE NEDERLAND V.o.F.**
**Rijksstraatweg 32**
**NL-9752 AE Haren(NL)**

(72) Inventor: **Roerdink, Eize**
**Laathofstraat 15**
**NL-6191 GD Beek(NL)**
Inventor: **Bruls, Wilhelmus Gerardus Marie**
**Graaf Wolterhoenstraat 32**
**NL-6243 BE Meerssen(NL)**
Inventor: **Steeman, Reinard Jozef Maria**
**J. Evertsenstraat 31**
**NL-6181 DD Stein(NL)**

(74) Representative: **Hoogstraten, Willem Cornelis Roeland et al**
**OCTROOIBUREAU DSM Postbus 9**
**NL-6160 MA Geleen(NL)**

## (54) New polymers, a process of their preparation and their use as stabilizers of aqueous formaldehyde solutions.

(57) The present invention is concerned with new polymers comprising about 7 to about 22 percent by weight vinyl acetate units, about 15 to about 35 percent by weight vinyl alcohol units about 35 to about 57 percent by weight vinyl acetal units, and about 7 to about 23 percent by weight of vinyl hemiacetal units.

These new polymers which differ from known polymers by the specific ranges of the acetate, alcohol and acetal units in combination with a hemiacetal content of about 7 to about 23 percent by weight show better solubilities in formaldehyde, and can be prepared with a higher degree of reproducibility than the prior art polymers.

EP 0 319 614 A1

# New polymers, a process of their preparation and their use as stabilizers of aqueous formaldehyde solutions

The invention relates to new polymers, a process for their preparation and their use as stabilizers of aqueous formaldehyde solutions.

The lifetime of aqueous formaldehyde solutions containing more than 37 % by weight formaldehyde is limited because of the precipitation of para-formaldehyde. Therefore, stabilizers have to be used to avoid the para-formaldehyde formation, or to decrease the storage temperature, thereby increasing the safety and avoiding the degradation into formic acid.

US-patent 4 247 487 describes aqueous solutions of formaldehyde stabilized against precipitation of para-formaldehyde by the incorporation therein of 0,1 to 1000 ppm of a low molecular weight vinyl polymer containing 1 to 40 mole percent of polyvinyl alcohol groups, 30 to 90 mole percent of polyvinyl acetal type groups and 0 to 30 mole percent polyvinyl acetate groups.

US-patent 4 085 079 describes concentrated aqueous formaldehyde solutions stabilized with a polymer containing from about 25 to about 90 parts by weight vinyl acetate units, from about 20 to about 72 parts by weight of vinyl acetal units and from about 3 to about 15 parts by weight of vinyl alcohol units.

As the stabilizing polymers of the US-patent 4 247 487 also the polymers of this patent are derived from a polyvinyl acetate of a relatively low weight average molecular weight (about 2000 to about 60000) by alcoholysis and acetalysation.

A drawback of the stabilizing polymers of the mentioned two patents resides in the fact that their solubility in formaldehyde is not very high, and that the reproducibility with which they can be prepared is varying.

Therefore, object of the present invention is the provision of polymers being suitable as stabilizers for aqueous formaldehyde solutions having a better solubility in formaldehyde than the known polymers which can be prepared with a high degree of reproducibility.

According to the present invention new polymers are provided comprising vinyl acetate units, vinyl alcohol units and vinyl acetal units, and being characterized by the fact that they contain about 7 to about 22 percent by weight vinyl acetate units, 15 to 35 % by weight vinyl alcohol units, 35 to 57 % by weight vinyl acetal units, and 7 to 23 % by weight vinyl hemiacetal units, the salt content being less than 10 % by weight in relation to the total solids content.

Surprisingly, these polymers being characterized by specific ranges of vinyl acetate units, vinyl alcohol units and vinyl acetal units in combination with a content of vinyl hemiacetal units within the specified range show a better solubility in formaldehyde than the known polymers used as stabilizers for aqueous formaldehyde solutions from which no content of vinyl hemiacetal units is reported.

Additionally, the present invention is based upon the discovery that the claimed polymers can be prepared in a reproducible manner if the known process (known from the US-patent 4 085 079) of preparing such polymers is carried out for a period of more than 4,5 hours.

Accordingly, the present invention is also concerned with a process for preparing the claimed polymers by alcoholizing and acetalyzing a polyvinyl acetate having a relatively low molecular weight in the presence of a strong acid catalyst at a temperature of approximately 40 to 65° C, neutralizing the acid catalyst by an alkaline material, and separating the formed salt. This process is characterized by the fact that the alcoholysis and acetalysation are carried out for a period of more than 4,5 hours.

Preferably the polymers of the present application have a weight average molecular weight in the range of about 2000 to about 60000, preferably of about 6000 to about 20000. They are readily dissolved in commercially available solvents, particularly methanol which is a usual component of aqueous formaldehyde solutions.

As already mentioned, the polymers of the present invention are prepared according to the process as disclosed in US-patent 4 085 079, with the exception that the duration of the alcoholysis and acetalysation is more than 4,5 hours. According to example 6 of US-patent 4 085 079 the longest duration of alcoholysis and acetalysation is 4,5 hours.

As the process of US-patent 4 085 079 the process used to prepare the polymers of the present invention is starting from a highly concentrated polyvinyl acetate solution (approximately 60 to 70 % by weight) whereas the process according to US-patent 4 247 487 uses a starting solution containing only about 5 % by weight polyvinyl acetate. This process insofar is disadvantageous as a considerable amount of the strong acid catalyst, for example sulfuric acid, is necessary, and the ratio of salt formed on neutralization to stabilizer is therefore rather high (2:1 to 3:1).

In generic terms the process for preparing the polymers of the present invention is carried out by dissolving the used polyvinyl acetate resins in a low boiling alcohol, for example methanol, adding acetaldehyde and subjecting the polyvinyl acetate to alcoholysis and acetalysation in presence of a strong acid catalyst having preferably a pKa at 25°C of at least about 2,0, for example a strong mineral acid, like sulfuric acid.

The alcoholysis and acetalysation are conveniently carried out concurrently, they may also be carried out sequentially.

After the alcoholysis and acetalysation are completed the reaction solution is neutralized with an alkaline material, for example with an alkali or alkaline earth metal oxide. hydroxide, carbonate, bicarbonate or a salt of low boiling organic acid being suitable to neutralize the strong acid catalyst. Potassium acetate is most preferably used as neutralizing agent.

Preferably the salt formed on neutralisation of the strong acid catalyst is separated from the reaction mixture by sedimentation as disclosed in the co-pending application

(application filed at the same date as the present application under the internal file number 5872).

According to a preferred embodiment of the process of the present application the reaction mixture after approximately two hours is stripped by means of an inert gas for example by nitrogen, preferably during a period of 10 to 20 minutes, to remove any excess of acetaldehyde.

After separation of the salt formed by neutralisation and purification and drying of the obtained polymer the same can be dissolved in any suitable solvent, for example lower aliphatic alcohols, ketons, amines, amides and ethers, to be added to the aqueous formed aldehyde solution which is to be stabilized. However it is also possible to add the polymers of the present invention in undissolved form because of the good solubility in formaldehyde.

The amounts in which the polymers of the present application are used to stabilize aqueous formaldehyde solutions depends on the formaldehyde concentration of the solutions to be stabilized. If the formaldehyde concentration of the aqueous solution is 37 to 70 percent by weight the polymers should be added in an amount of about 3 to about 50 ppm.

The following examples are set force to illustrate the invention but are not to be construed as limiting the scope thereof.

## Example 1

Into a reaction vessel containing 300 g methanol (water content less than 0,3 percent by weight) which has been purged with nitrogen 667 g polyvinyl acetate having an average molecular weight of approximately 15000 (VINNAPAS B 1,5, Wacker Chemie) are added during 2,5 hours under stirring at room temperature. After the polyvinyl acetate has been dissolved the solution is still stirred to obtain a homogenized solution.

Then 200 g acetaldehyde are pumped into the closed reactor. Subsequently under cooling 61,8 g sulfuric acid (96 %) in 53,4 methanol are added to the reactor.

Then the reaction mixture is heated to the reaction temperature of 60 ± 1°C and reacted under stirring during a period of 2 to 10 hours.

After the alcoholysis and acetalysation have been completed to the cooled reaction mixture 118,6 g potassium acetate in 560 g methanol are pumped into the reactor.

The neutralized reaction mixture after an additional stirring period of 3 minutes is allowed to sedimentate overnight and the supernatant solution is centrifuged.

The separated quaterpolymers being obtained during different reaction times varying between 2 and 10 hours are analyzed, and the obtained results are summarized in the following table I.

## Table I

### Quaterpolymer properties

| Experiment Nr. | | Reaction-time, (hrs) | Hemiacetal mole%** | Hemiacetal wt.% | Acetal mole%*** | Acetal wt.% | Total Acetal-content mole% | Total Acetal-content wt.% | VAc units mole% | VAc units wt.% | VAl units mole% | VAl units wt.% |
|---|---|---|---|---|---|---|---|---|---|---|---|---|
| Comparison Ex.[1] | 1 | 2 | 5,0 | 6,3 | 33,5 | 27,5 | 38,5 | 33,8 | 45,0 | 55,7 | 16,5 | 10,5 |
| Comparison Ex.[1] | 2 | 4 | 7,0 | 9,9 | 48,5 | 44,4 | 55,5 | 54,3 | 21,0 | 29,0 | 23,5 | 16,6 |
| Ex. | 1 | 6 | 9,0 | 13,5 | 53,5 | 51,5 | 62,0 | 65,0 | 9,0 | 13,2 | 29,0 | 21,8 |
| Ex. | 2 | 8 | 10,5 | 16,1 | 51,0 | 50,7 | 61,5 | 66,8 | 5,0 | 7,5 | 33,5 | 25,7 |
| Ex. | 3 | 10 | 11,5 | 17,0 | 51,0 | 51,1 | 62,0 | 68,1 | 3,5 | 5,3 | 34,5 | 26,7 |

[1] outside of scope of protection

The properties of the methanolic quaterpolymer-solutions are summarized in the following table II:

Table II

Solution properties

| Experiment No. | | Reaction-time, (hrs) | TSC*(%) | ash content (%) |
|---|---|---|---|---|
| Comparison Ex. | 1 | 2 | 35,2 | 1,9 |
| Comparison Ex. | 2 | 4 | 32,2 | 1,9 |
| Ex. | 1 | 6 | 30,7 | 1,9 |
| Ex. | 2 | 8 | 31,2 | 2,0 |
| Ex. | 3 | 10 | 31,8 | 2,0 |

*TSC = Total solids content

**definition of mole% hemiacetal = no. of

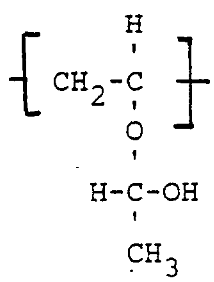

units / original number of vinylacetate units

***definition of mole% acetal = $\frac{1}{2}$ . no. of

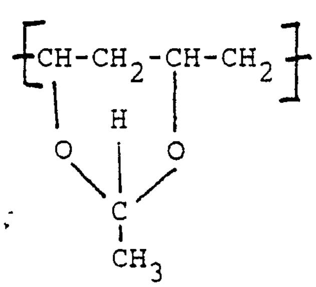

units / original number of vinylacetate units

Example 2

This example is performed according to example 1, however, after 2 hours of acetalysation the reactor content is stripped with nitrogen during 10 minutes in order to remove the excess of acetaldehyde.

After stripping the reaction is continued for another 4 hours. To test the reproducibility 8 charges are prepared.

The quaterpolymer properties and the solution properties are summarized in table III:

Table III

| | Quaterpolymer properties | | | Solution properties | |
|---|---|---|---|---|---|
| | Hemiacetal mole% | Acetal mole% | Total Acetal mole% | TSC wt.% | ash content wt.% |
| Charge 1 | 7,0 | 53 | 60 | 31,1 | 1,99 |
| Charge 2 | 6,7 | 53,3 | 60 | 31,7 | 2,09 |
| Charge 3 | 8,0 | 53 | 61 | 31,5 | 2,00 |
| Charge 4 | 7,8 | 53,2 | 60 | 36,1 | 2,36 |
| Charge 5 | 8,3 | 52,7 | 61 | 31,7 | 2,06 |
| Charge 6 | 8,3 | 52,7 | 61 | 31,0 | 2,11 |
| Charge 7 | 7,4 | 52,6 | 60 | 30,9 | 2,05 |
| Charge 8 | 7,8 | 52,2 | 60 | 30,0 | 2,03 |

The figures in table III show that the polymers of the present invention can be produced with a high degree of reproducibility.

Example 3

This example is performed according to example 1 and the acetalysation time amounts to 6 hours. The results are presented in tables IV and V:

Table IV

Quaterpolymer properties

| Experiment | Hemiacetyl | | Acetal | | Total Acetalysation | | VAc units | | VAl units | |
|---|---|---|---|---|---|---|---|---|---|---|
| | mole% | wt.% | mole% | wt.% | mole% | wt.% | mole% | wt.% | mole% | wt.% |
| 1 | 10 | 14,9 | 52,5 | 50,5 | 62,5 | 65,3 | 9,5 | 13,8 | 28 | 20,8 |

Table V

Solution properties

| TSC, wt.% | ash content, wt.% |
|---|---|
| 28 | 2,0 |

The quaterpolymer is added as stabilizer to a 45 percent by weight formaldehyde solution in water in an amount of 10 mg per kg of the formaldehyde solution which is stored at 22,5° C.

The solution remains clear up to 72 days storage.

A comparison experiment without addition of stabilizer shows that the formaldehyde solution became turbid under the same conditions within 0,5 hours.

Example 4

This example shows the good solubilities in formaldehyde of the quaterpolymers of experiments 1 to 3 in comparison to the quaterpolymers of the comparison experiments 1 and 2 which are outside of the scope of invention, measured by extinction tests which are carried out as follows:

A solution of the polymer in methanol, containing 1 percent by weight of the polymer, is gently injected to a mildly stirred, clear and colourless aqueous formaldehyde solution containing 45 percent by weight of formaldehyde, not more than 1,5 percent by weight of methanol and not more than 0,04 percent by weight of formic acid, the aqueous formaldehyde solution having a temperature of 70 degrees centigrade, in such quantity that the polymer concentration in the aqueous formaldehyde solution becomes 0,015 percent by weight.

After injection of the polymer solution the resulting extinction at about 400 nm is measured photometrically in comparison to the extinction before injection.

The extinction is a measure for the degree of turbidity, caused by the injection of the polymer solution to the aqueous formaldehyde solution. The injected quantity of the polymer solution is about 10 to 20 times greater than normally necessary to stabilize the aqueous formaldehyde solution.

The stated extinction values are the measured values, multiplied by 1000. A low extinction value indicates a good solubility.

Table VI

| Experiment | Extinction |
|---|---|
| Comparison Ex. 1 | 45 |
| Comparison Ex. 2 | 30 |
| Ex. 1 | 20 |
| Ex. 2 | 19 |
| Ex. 3 | 25 |

The figures in the above table clearly show the good solubilities of the claimed quaterpolymers.

Example 5

Into a reactor 1,35 parts by weight methanol are introduced. Then 245 parts by weight polyvinyl acetate having a weight average molecular weight of 15000 are dissolved in the methanol under stirring until the dissolution is completed. Under colling 0,23 parts by weight sulfuric acid (98 % by weight $H_2SO_4$) are dissolved in 0,2 parts by weight methanol. During the dissolution the temperature is maintained below 35 °C.

Thereupon 0,73 parts by weight acetaldehy e are added to the reactor. Subsequently the sulfuric acid/methanol-mixture is added to the reactor. The reactor content is heated to 60 °C. During 2 hours the reactor content is maintained at 60 °C while it is intensively stirred. Next the reactor content is stripped by means of nitrogen (15 l $N_2$/l reactor volume) during about 15 minutes. During stripping the reaction temperature is maintained constant. After stripping the reaction is allowed to continue for 4 hours at 60 °C. The reaction is stopped by adding to the reactor a solution of 0,44 parts by weight potassium acetate in 2,13 parts by weight methanol.

During the neutralization with potassium acetate a very finely divided precipitate of potassium sulfate is formed (particle size < 3 µm). This salt cannot be separated from the reaction mixture by filtration. The whole reaction mixture is transferred to a sedimentation column in which the separation of the salt from the reaction product takes place by sedimentation under influence of gravity. It has been found that with a liquid height of 0,5 to 0,6 m a good separation can be obtained in 4 days when a sedimentation column having a H/D-ratio of 1 is used. The clear supernatant is drawn off after 4 days and filtered to remove any suspended impurities. After dilution the solution is ready for use.

## Claims

1. Polymers comprising vinyl acetate units, vinyl alcohol units and vinyl acetal units, characterized in that they contain about 7 to about 22 percent by weight vinyl acetate units, about 15 to about 35 percent by weight vinyl alcohol units, about 35 to about 57 percent by weight vinyl acetal units, and about 7 to about 23 percent by weight of vinyl hemiacetal units, the salt content being less than 10 percent by weight in relation to the total solids content.

2. Polymers according to claim 1, characterized in that their weight average molecular weight ranges between about 2000 and about 60000, preferably between about 6000 and about 20000.

3. A process for preparing the polymers of the claims 1 and 2 by alcoholysing and acetalysing a polyvinyl acetate having a relatively low molecular weight in the presence of a strong acid catalyst at a temperature of approximately 40 to 65 °C, neutralizing the acid catalyst by an alkaline material, and separating the formed salt, characterized in that the alcoholysis and the acetalysation are carried out for a period of more than 4,5 hours.

4. A process according to claim 3, characterized in that a polyvinyl acetate solution is used containing 60 to 70 percent by weight polyvinyl acetal.

5. The use of the polymers of the preceding claims for stabilizing an aqueous formaldehyde solution.

European Patent Office

# EUROPEAN SEARCH REPORT

Application Number

EP 87 20 2434

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US-A-4 247 487 (J.S. PERCY)<br>* Claims 1-7 * | 1 | C 08 F 8/28<br>C 08 F 8/12<br>C 07 C 47/04 |
| D,A | US-A-4 085 079 (R.C. KMETZ)<br>* Claims 1-9 * | 1 | |
| A | GB-A- 856 840 (WACKER-CHEMIE)<br>* Claims 1-11; example * | 1 | |
| A | GB-A-1 199 652 (UNION CARBIDE CORP.)<br>* Claims 1-6 * | 1 | |
| A | DE-A- 755 027 (SOCIETE NOBEL FRANCAISE, PARIS)<br>* Claim * | 1 | |
| | | | TECHNICAL FIELDS SEARCHED (Int. Cl.4) |
| | | | C 08 F |

The present search report has been drawn up for all claims

| Place of search | Date of completion of the search | Examiner |
|---|---|---|
| THE HAGUE | 26-08-1988 | PERMENTIER W.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)